# EUROPÄISCHE PATENTSCHRIFT

(11) **EP 3 090 693 B1**
(45) Veröffentlichungstag und Bekanntmachung des Hinweises auf die Patenterteilung: **12.06.2019**
(21) Anmeldenummer: 16163820.0
(22) Anmeldetag: 05.04.2016
(51) Int. Cl.: A61B 17/14, A61B 17/15

(54) **CHIRURGISCHES INSTRUMENT ZUR WEKRZEUGFÜHRUNG**
SURGICAL TOOL GUIDE
GUIDE D'OUTIL CHIRURGICAL

(30) Priorität: 29.04.2015 DE 102015106655
(43) Veröffentlichungstag der Anmeldung: 09.11.2016
(73) Patentinhaber: Aesculap AG, 78532 Tuttlingen (DE)
(72) Erfinder: Weiß, Josef-Benedikt, 78628 Rottweil (DE); Utz, Michael, 78532 Tuttlingen (DE)
(74) Vertreter: Hoeger, Stellrecht & Partner Patentanwälte mbB

(56) Entgegenhaltungen:
- US-A- 5 490 854
- US-A- 5 611 802
- US-A1- 2005 240 195
- US-A1- 2013 325 017
- US-B1- 7 182 766

## Beschreibung

Die Erfindung betrifft ein chirurgisches Instrument zur Werkzeugführung.

Ein chirurgisches Instrument zur Werkzeugführung kommt zum Führen eines chirurgischen Werkzeugs zum Einsatz und umfasst zu diesem Zweck mindestens ein Führungselement, in welches das Werkzeug eingreifen und von dem das Werkzeug geführt werden kann. Dies erleichtert einem Operateur die Handhabung des Werkzeugs und erlaubt eine definierte Bearbeitung des Körperteils, an dem das Werkzeug zum Einsatz kommt und an welchem das Instrument vorzugsweise während der Bearbeitung festgelegt ist.

Ein Beispiel für ein Instrument zur Werkzeugführung ist ein chirurgischer Sägeblock, der beispielsweise während einer Knieoperation beim Präparieren des Femurs oder der Tibia zum Einsatz kommt. Der Sägeblock wird zum Beispiel distal am Femur fixiert, und ein Werkzeug in Gestalt eines chirurgischen Sägeblattes kann vom mindestens einen Führungselement geführt werden.

In der US 2013/0325017 A1 ist ein Instrument zur Werkzeugführung offenbart, bei dem zwischen seitlich nebeneinander angeordneten Komponenten schlitzförmige Führungselemente gebildet sind. Am Rand des Instrumentes sind die Komponenten mit klammerartigen Halterungen zusammengehalten, zwischen denen sich die Führungselemente erstrecken.

Aufgabe der vorliegenden Erfindung ist es, ein chirurgisches Instrument zur Werkzeugführung bereitzustellen, das einem Operateur die Handhabung des geführten Werkzeugs erleichtert und nach Möglichkeit kostengünstig herstellbar ist.

Diese Aufgabe wird erfindungsgemäß durch ein chirurgisches Instrument zur Werkzeugführung mit den Merkmalen von Anspruch 1 gelöst.

Bei dem erfindungsgemäßen Instrument kann das Werkzeug von mindestens einem Führungselement geführt werden, wobei zwei Führungselemente vorgesehen sind, die nicht miteinander verbunden sind oder ineinander münden. Zu diesem Zweck kann es in einer Eingriffsrichtung in das Führungselement eingeführt werden und in dieses eingreifen und insbesondere den Grundkörper durchgreifen. Im Winkel und insbesondere quer zur Eingriffsrichtung erstreckt sich das Führungselement bis zu einem Rand des Grundkörpers, beispielsweise einer Seitenfläche oder einer Seitenwand des Grundkörpers. Auf diese Weise weist das Führungselement eine seitliche Öffnung am Rand des Grundkörpers auf. Dies erlaubt es zum Beispiel, den Grundkörper herstellungstechnisch einfach und kostengünstig aus einem Kunststoffmaterial zu fertigen, zum Beispiel als Kunststoffformteil. Eine entsprechende Form kann konstruktiv einfach beschaffen sein, damit das sich bis zum Rand des Grundkörpers erstreckende Führungselement gebildet werden kann. Insbesondere ist ein Bereitstellen des Instrumentes oder des Grundkörpers für die einmalige Verwendung denkbar (Single Use). Das mindestens eine Blockierelement ist vorgesehen, um einem Operateur die Handhabung des Instrumentes und das Führen des Werkzeugs zu erleichtern, wenn das mindestens eine Blockierelement die Blockierstellung einnimmt. In der Blockierstellung bildet das mindestens eine Blockierelement einen Anschlag für das Werkzeug am mindestens einen Führungselement. Das Werkzeug kann dadurch nicht über die seitliche Öffnung am Rand aus dem Führungselement austreten. Dies erleichtert einem Benutzer das Führen des Werkzeuges. Insbesondere kann der beim Bearbeiten eines Körperteils vom Werkzeug erfasste Bereich durch das Blockierelement in dessen Blockierstellung eingeschränkt werden. Beispielsweise erweist sich dies als vorteilhaft, wenn das Instrument ein beim Bearbeiten des Femurs oder der Tibia eingesetzter chirurgischer Sägeblock ist und die seitliche Öffnung lateral oder medial am Sägeblock angeordnet ist. Bei der Knieoperation nimmt das Kniegelenk beim eröffneten Zugang eine abgewinkelte Stellung ein, wobei die Seitenbänder am Femur und an der Tibia fixiert bleiben. Indem der Austritt des Werkzeugs lateral bzw. medial mittels des Blockierelementes verhindert wird, kann die Gefahr eines unerwünschten Kontaktes des Werkzeuges mit den Seitenbändern verringert und eine Verletzung der Seitenbänder weitgehend vermieden werden.

Wie bereits erwähnt kann das Instrument ein chirurgischer Sägeblock sein und das mindestens eine Führungselement eine Führung für ein chirurgisches Sägeblatt.

Der Grundkörper ist einstückig.

Mindestens ein Führungselement kann schlitzförmig ausgestaltet sein, beispielsweise zum Führen eines chirurgischen Sägeblattes.

Der Grundkörper ist vorzugsweise aus einem Kunststoffmaterial gefertigt und/oder als Kunststoffformteil gebildet. Dies erlaubt es, die Herstellungskosten für das Instrument gering zu halten, wodurch eine Einmalverwendung des Grundkörpers oder des Instrumentes begünstigt wird.

Am Grundkörper können bei einer vorteilhaften Ausführungsform des Instrumentes zwei oder mehr Führungselemente gebildet sein, wobei das mindestens eine Blockierelement einen jeweiligen Anschlag an zwei oder mehr Führungselementen bildet. Mit einem Blockierelement ist dadurch die Möglichkeit gegeben, einen Anschlag an zwei oder mehr Führungselementen für das Werkzeug zu bilden. Kommen bei einem Einsatz des Instrumentes zwei oder mehr Führungselemente zum abwechselnden oder aufeinanderfolgenden Führen des Werkzeugs zum Einsatz, bedarf es nur eines Blockierelementes, um die vorstehend erwähnten Vorteile zu erzielen. Überdies kann die konstruktive Ausgestaltung eines Instrumentes mit zwei oder mehr Führungselementen vereinfacht werden.

Bei einer vorteilhaften Ausführungsform des Instrumentes durchsetzt das mindestens eine Blockierelement mindestens ein Führungselement in der Blockierstellung. Beispielsweise ist das Blockierelement quer zu einer von einem als Führungsschlitz ausgestalteten Führungselement definierten Ebene ausgerichtet.

Das mindestens eine Blockierelement kann beispielsweise stiftförmig sein.

Insbesondere bei der zuletzt erwähnten vorteilhaften Ausführungsform kann das mindestens eine Blockierelement als Schraube ausgestaltet sein, beispielsweise als Knochenschraube. Die Schraube ist bei Einnahme der Blockierstellung beispielsweise mit dem Grundkörper verschraubt und dadurch an diesem gesichert.

Das mindestens eine Blockierelement kann in der Blockierstellung im Abstand zum Rand angeordnet sein.

Denkbar ist auch, dass das mindestens eine Blockierelement die seitliche Öffnung mindestens eines Führungselementes in der Blockierstellung zumindest teilweise überdeckt. Zu diesem Zweck kann das Blockierelement insbesondere eine Abdeckung der mindestens einen seitlichen Öffnung bilden oder umfassen.

Beispielsweise ist das mindestens eine Blockierelement, etwa bei der zuletzt erwähnten vorteilhaften Ausgestaltung, plattenförmig oder streifenförmig ausgestaltet.

Das mindestens eine Blockierelement kann getrennt vom Grundkörper gebildet sein und an diesem zur Einnahme der Blockierstellung festlegbar sein. Dadurch weist das Instrument eine höhere Vielseitigkeit auf. Der Operateur kann das Blockierelement bedarfsabhängig in die Blockierstellung überführen, sofern dies erforderlich ist.

Es kann bei einer andersartigen Ausführungsform vorgesehen sein, dass das mindestens eine Blockierelement am Grundkörper gehalten ist, insbesondere dass das mindestens eine Blockierelement in der Blockierstellung vormontiert ist. Beispielsweise ist das Blockierelement durch Vormontage werksseitig am Grundkörper gehalten. Der Operateur kann das mindestens eine Blockierelement bedarfsabhängig vom Grundkörper lösen, falls er dessen nicht bedarf.

Das mindestens eine Blockierelement kann mit dem Grundkörper zum Beispiel, vorzugsweise über eine Sollbruchverbindung, verbunden und vom Grundkörper lösbar sein. In einem Auslieferungszustand ist das Blockierelement am Grundkörper gehalten. Durch Lösen der Verbindung kann der Operateur, wenn er des Blockierelementes nicht bedarf, dieses vom Grundkörper lösen.

Von Vorteil ist es, wenn das mindestens eine Blockierelement ausgehend von der Blockierstellung in eine Freigabestellung überführbar ist, in der die seitliche Öffnung für das Werkzeug freigegeben ist.

Es kann vorgesehen sein, dass das mindestens eine Blockierelement am Grundkörper schwenkbar und/oder verschieblich gelagert ist und von einer Freigabestellung, in der die seitliche Öffnung für das Werkzeug freigegeben ist, in die Blockierstellung und vorzugsweise auch umgekehrt überführbar ist. Beispielsweise ist das mindestens eine Blockierelement am Grundkörper gehalten oder werksseitig vormontiert bereitgestellt, wobei das Blockierelement eine Freigabestellung einnimmt. Bei Bedarf kann der Operateur das Blockierelement in die Blockierstellung überführen und dadurch den Anschlag für das Werkzeug bilden.

Das Blockierelement kann beispielsweise einstückig mit dem Grundkörper verbunden sein. Denkbar ist es, dass das Blockierelement mittels eines Filmscharniers schwenkbar mit dem Grundkörper verbunden ist.

In der Blockierstellung ist das Blockierelement am Grundkörper vorzugsweise kraftschlüssig und/oder formschlüssig fixiert, beispielsweise klemmend und/oder rastend. Der Grundkörper und das mindestens eine Blockierelement können korrespondierende und zusammenwirkende Klemmelemente und/oder Rastelemente umfassen oder bilden.

Das Blockierelement kann mit dem Grundkörper in der Blockierstellung beispielsweise verschraubt sein.

Von Vorteil ist es, wenn das mindestens eine Blockierelement am Grundkörper wahlweise in einer Mehrzahl von Blockierstellungen festlegbar ist. Dadurch wird die Vielseitigkeit des Instrumentes erhöht. Je nach Bedarf kann der Operateur wählen, welche der Blockierstellungen das mindestens eine Blockierelement am Grundkörper einnimmt. Dadurch kann Einfluss auf den Bewegungsumfang des Werkzeuges relativ zum Grundkörper in mindestens einem Führungselement genommen werden.

Es kann vorgesehen sein, dass am Grundkörper eine Mehrzahl von Aufnahmen gebildet ist, in die das mindestens eine Blockierelement zur Einnahme unterschiedlicher Blockierstellungen wahlweise einführbar ist. Zum Beispiel sind quer und/oder längs der Eingriffsrichtung eine Mehrzahl von Aufnahmen für das Blockierelement angeordnet, deren eine der Operateur auswählen kann.

Alternativ oder ergänzend kann vorgesehen sein, dass das mindestens eine Blockierelement in einer am Grundkörper gebildeten Aufnahme in einer jeweiligen Blockierstellung festlegbar beweglich ist. Beispielsweise ist die Aufnahme quer und/oder längs zur Eingriffsrichtung erstreckt, und das Blockierelement kann in unterschiedlichen Relativpositionen zum Grundkörper in der Aufnahme fixiert werden und dadurch unterschiedliche Blockierstellungen einnehmen.

Bei den beiden zuletzt erwähnten vorteilhaften Ausführungsformen kann über das mindestens eine Blockierelement der Bewegungsumfang des Werkzeugs in einem Führungselement beeinflusst werden. Dies umfasst auch einen möglichen Winkel, den das Werkzeug mit der Eingriffsrichtung einnimmt.

Die nachfolgende Beschreibung bevorzugter Ausführungsformen der Erfindung dient im Zusammenhang mit der Zeichnung der näheren Erläuterung der Erfindung. Es zeigen:
- Figur 1:: eine perspektivische Darstellung einer ersten bevorzugten Ausführungsform eines erfindungsgemäßen Instrumentes;
- Figur 2:: eine Darstellung in Blickrichtung "A" in Figur 1;
- Figur 3:: eine perspektivische Darstellung einer zweiten bevorzugten Ausführungsform eines erfindungsgemäßen Instrumentes;
- Figur 4:: das Instrument in Blickrichtung "B" in Figur 3;
- Figur 5:: eine perspektivische Darstellung, in der zwei weitere vorteilhafte Ausführungsformen des erfindungsgemäßen Instrumentes teilweise gezeigt sind, wobei Blockierelemente der Instrumente eine Freigabestellung einnehmen und
- Figur 6: eine Darstellung entsprechend Figur 5, wobei die Blockierelemente eine Blockierstellung einnehmen.

Bei den nachfolgend erläuterten vorteilhaften Ausführungsformen des erfindungsgemäßen Instrumentes werden gleiche oder gleichwirkende Merkmale oder Bauteile mit identischen Bezugszeichen gekennzeichnet.

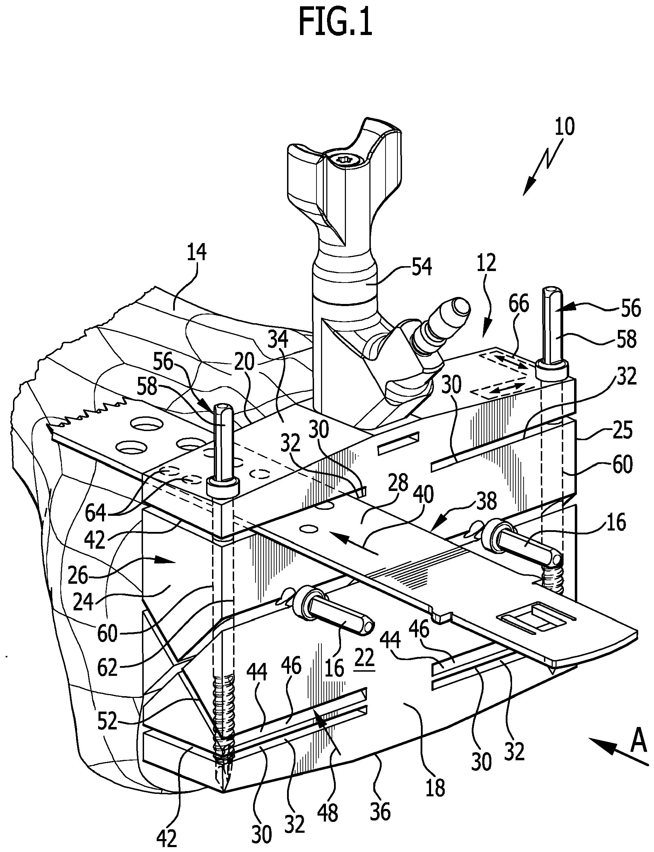

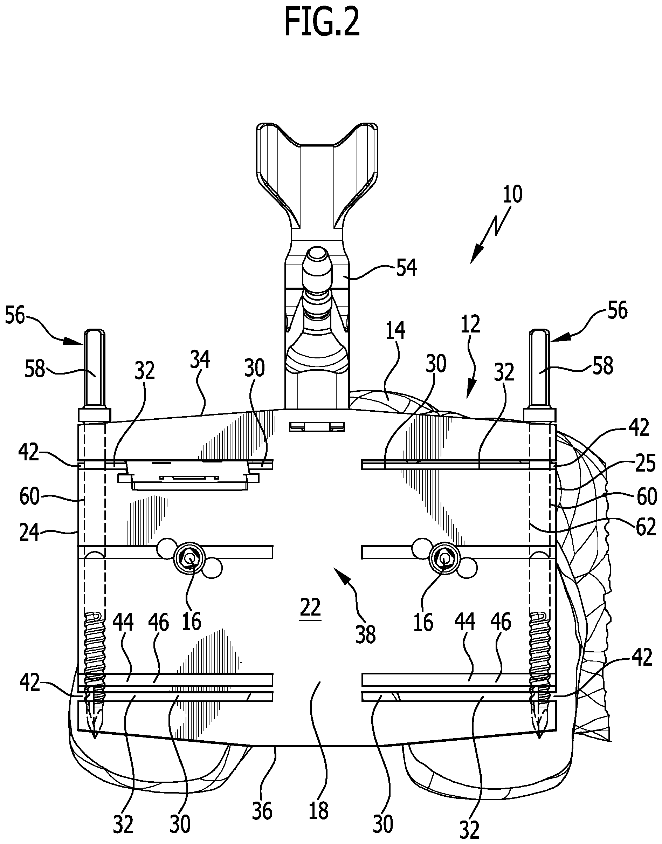

Die Figuren 1 und 2 zeigen eine mit dem Bezugszeichen 10 belegte erste vorteilhafte Ausführungsform eines erfindungsgemäßen chirurgischen Instrumentes zur Werkzeugführung. Das Instrument 10 ist ein chirurgischer Sägeblock 12. Der Sägeblock 12 kommt beispielsweise bei der Präparation eines Femurs 14 bei einer Knieoperation zum Einsatz. Zu diesem Zweck wird der Sägeblock 12 zum Beispiel mittels Fixierelementen 16, vorliegend Knochenschrauben, distal am Femur 14 festgelegt.

Das Instrument 10 umfasst einen Grundkörper 18. Der Grundkörper 18 weist vorliegend eine im Wesentlichen quaderförmige Gestalt auf und umfasst eine erste Seite 20, die im bestimmungsgemäßen Gebrauch dem distalen Ende des Femurs 14 zugewandt ist. Gegenüberliegend weist der Grundkörper 18 eine zweite Seite 22 auf, die dem Operateur zugewandt ist. Die erste Seite 20 und die zweite Seite 22 sind über einander gegenüberliegende Seitenflächen 24 und 25 des Grundkörpers 18 miteinander verbunden. Die Seitenflächen 24, 25 sind an Schmalseiten des Grundkörpers 18 angeordnet und Bestandteil eines Randes 26 des Grundkörpers 18.

Beim Einsatz des Sägeblocks 12 zum Präparieren des Femurs 14 kommt ein chirurgisches Werkzeug zum Einsatz. Das Werkzeug ist vorliegend ein chirurgisches Sägeblatt 28, das in eine in der Zeichnung nicht dargestellte chirurgische Säge eingespannt und von dieser antreibbar ist.

Zum Führen des Sägeblattes 28 weist der Grundkörper 18 eine Mehrzahl von Führungselementen 30 auf. Die Führungselemente 30 sind vorliegend Führungsschlitze 32.

Insgesamt sind vier Führungsschlitze 32 vorgesehen. Zwei Führungsschlitze 32 erstrecken sich von der Seitenfläche 24 vom Rand 26 in Richtung der gegenüberliegenden Seitenfläche 25. Die Schlitze sind quer zur ihrer Erstreckung voneinander beabstandet, wobei ein erster Führungsschlitz 32 nahe einer oberen Seite 34 und ein zweiter Führungsschlitz 32 nahe einer unteren Seite 36 des Grundkörpers 18 angeordnet ist.

Von der Seitenfläche 25 erstrecken sich ebenfalls zwei Führungsschlitze 32, in Richtung der Seitenfläche 24. Die Führungsschlitze 32 sind voneinander beabstandet, wobei auch in diesem Fall ein Führungsschlitz 32 nahe der oberen Seite 34 und ein weiterer Führungsschlitz 32 nahe der unteren Seite 36 angeordnet ist.

Die nahe der oberen Seite 34 angeordneten Führungsschlitze 32 definieren eine gemeinsame Ebene. Ebenso definieren die nahe der unteren Seite 36 angeordneten Führungsschlitze 32 eine gemeinsame Ebene. Die jeweils in der gemeinsamen Ebene verlaufenden Führungsschlitze 32 sind nicht miteinander verbunden oder münden nicht ineinander. In einem mittleren Bereich 38 des Grundkörpers 18, mittig zwischen den Seitenflächen 24 und 25, ist keiner der Führungsschlitze 32 erstreckt.

Die Führungsschlitze 32 durchsetzen den Grundkörper 18 von der zweiten Seite 22 zur ersten Seite 20. Dies entspricht einer Eingriffsrichtung 40 des Sägeblattes 28 und insbesondere einer Durchgriffsrichtung des Sägeblattes 28 durch den Grundkörper 18, wenn das Instrument 10 bestimmungsgemäß zum Einsatz kommt (Figur 1). Das Sägeblatt 28 kann in der Eingriffsrichtung 40 in die Schlitze 32 eingeführt werden.

Die Führungsschlitze 32 sind dementsprechend quer zur Eingriffsrichtung 40 bis zum Rand 26 erstreckt. Am Rand 26 sind die Führungsschlitze 32 mit einer jeweiligen seitlichen Öffnung 42 seitlich geöffnet.

Im Grundkörper 18 sind zwei weitere Führungselemente 44 gebildet, die ebenfalls ausgestaltet sind als Führungsschlitze 46 für das Sägeblatt 28. Die Führungsschlitze 46 definieren Ebenen, die im Winkel zu den von den Führungsschlitzen 32 definierten Ebenen ausgerichtet sind. An der zweiten Seite 22 sind Öffnungen der Führungsschlitze 46 nahe den Öffnungen der Führungsschlitze 32 an der unteren Seite 36 angeordnet. An der ersten Seite 20 sind Öffnungen der Führungsschlitze 46 ungefähr mittig zwischen der oberen Seite 34 und der unteren Seite 36 positioniert. Die Führungsschlitze 46 sind dadurch von der Seite 22 zur Seite 20 von unten nach oben geneigt ausgestaltet.

Die Führungsschlitze 46 erstrecken sich quer zu einer Eingriffsrichtung 48 (in der Ebene der Führungsschlitze 46) des Sägeblattes 28 bis zum Rand 26 an den Seitenflächen 24, 25. Am Rand 26 sind die Führungsschlitze 46 über seitliche Öffnungen 52 seitlich geöffnet. In der Eingriffsrichtung 48 kann das Sägeblatt 28 in die Schlitze 46 eingeführt werden.

Das Vorsehen der seitlichen Öffnungen 42, 52 der Führungsschlitze 32 bzw. 46 erweist sich als vorteilhaft für die Herstellung des Grundkörpers 18. Insbesondere ist es möglich, den Grundkörper 18 als Kunststoffformteil herstellungstechnisch einfach und kostengünstig zu fertigen. Auch die entsprechende Form für die Herstellung kann konstruktiv einfach beschaffen sein. Die kostengünstige Herstellung des Grundkörpers 18 und damit des Instrumentes 10 gibt insbesondere die Möglichkeit, dieses als einmal verwendbares (Single Use) Instrument bereitzustellen.

Am Grundkörper 18 ist an der oberen Seite 34 in Verlängerung des mittleren Bereiches 38 ein Halteelement 54 angeordnet. Das Halteelement 54 dient zur Aufnahme einer in der Zeichnung nicht dargestellten chirurgischen Markiereinrichtung. Mit einem chirurgischen Navigationssystem, ebenfalls nicht dargestellt, kann die Markiereinrichtung und damit das Instrument 10 im Raum verfolgt werden. Die Lage und die Orientierung des Instrumentes 10 lassen sich auf diese Weise vom Navigationssystem ermitteln, um den Femur 14 navigationsgestützt zu präparieren.

Es kann vorgesehen sein, dass das Halteelement 54 bereits bei der Herstellung des Grundkörpers 18 an diesen angeformt wird. Denkbar ist auch, dass das Halteelement 54 nachträglich mit dem Grundkörper 18 verbunden wird. Beispielsweise wird das Halteelement 54 mit dem Grundkörper 18 verschraubt.

Wie erwähnt kann mit dem Grundkörper 18 das Sägeblatt 28 bei der Bearbeitung des Femurs 14 über den jeweils ausgewählten Führungsschlitz 32, 46 geführt werden (Figuren 1 und 2).

Um zu vermeiden, dass das Sägeblatt 28, insbesondere unbeabsichtigt, über die jeweilige seitliche Öffnung 42, 52 aus dem Führungsschlitz 32 bzw. 46 austritt, umfasst das Instrument 10 mindestens ein Blockierelement 56. Vorliegend sind zwei Blockierelemente 56 vorgesehen, die nahe den Seitenflächen 24, 25 am Grundkörper 18 festgelegt werden können. Die Blockierelemente 56 sind insbesondere identisch ausgestaltet. Vorzugsweise sind die Blockierelemente 56 stiftförmig, insbesondere sind die Blockierelemente 56 Knochenschrauben 58.

Am Grundkörper 18 ist für das Blockierelement 56 vorzugsweise jeweils eine Aufnahme 60 gebildet. Die Aufnahme 60 kann beispielsweise als Kanal oder Sackloch 62 im Grundkörper 18 ausgestaltet sein, zum Beispiel über eine Bohrung.

Im vorliegenden Fall ist die Aufnahme 60 quer zu den von den Führungsschlitzen 32 definierten Ebenen ausgerichtet und erstreckt sich von der oberen Seite 34 bis nahe der unteren Seite 36. Die Aufnahme 60 ist nahe der zweiten Seite 22 am Rand 26 angeordnet, wobei jeder Seitenfläche 24, 25 und dementsprechend dem Blockierelement 56 eine Aufnahme 60 zugeordnet ist.

Nimmt das Blockierelement 56 eine Blockierstellung ein, wirkt es als Anschlag für das Sägeblatt 28. Dies verhindert ein Austreten des Sägeblattes 28 aus dem Führungsschlitz 32 oder 46 über die jeweilige seitliche Öffnung 42 bzw. 52. Dadurch wird der Bewegungsumfang des Sägeblattes 28 relativ zum Grundkörper 18 quer zur Eingriffsrichtung 40 bzw. 48 begrenzt. Insbesondere wird verhindert, dass das Sägeblatt 28 an den Seitenflächen 24, 25 aus dem Grundkörper 18 austritt. Auf diese Weise werden Seitenbänder des Kniegelenkes (in der Zeichnung nicht dargestellt), die bei der Präparation des Femurs 14 freigelegt sind, vor Verletzung durch das Sägeblatt 28 geschützt. Überdies wird dem Operateur die Führung des Sägeblattes 28 erleichtert.

Im vorliegenden Fall nimmt die jeweilige Knochenschraube 58 die Blockierstellung ein, wenn sie in der Aufnahme 60 mit dem Grundkörper 18 verschraubt und dadurch an diesem fixiert ist. Es kann vorgesehen sein, dass das Überführen der Knochenschraube 58 in die Blockierstellung durch den Operateur durchgeführt wird, sofern dieser den Einsatz eines Blockierelementes 56 wünscht. Denkbar ist auch, dass die Knochenschrauben 58 bereits werksseitig mit dem Grundkörper 18 verschraubt und dadurch das Instrument 10 mit die Blockierstellung einnehmendem Blockierelement 56 bereitgestellt werden.

Der Operateur kann die Knochenschrauben 58 vom Grundkörper 18 lösen, sofern der Einsatz eines Blockierelementes 56 nicht erforderlich ist. Das Instrument erweist sich dadurch als besonders vielseitig.

Figur 1 zeigt an der oberen Seite 34 nahe der Seitenfläche 24 schematisch eine Mehrzahl von Eintrittsöffnungen 64, die gestrichelt dargestellt sind. Hierbei handelt es sich um Eintrittsöffnungen 64 von Aufnahmen, die im Grundkörper 18 ergänzend oder alternativ zur Aufnahme 60 gebildet sein könnten. Die zusätzlichen Aufnahmen selbst sind nicht gezeigt. Insbesondere verlaufen diese Aufnahmen parallel zur Aufnahme 60. Der Operateur kann wahlweise eine der Aufnahmen zur Positionierung der Knochenschraube 58 auswählen. Dies gibt die Möglichkeit, dass die Knochenschraube 58 unterschiedliche Blockierstellungen am Grundkörper 18 einnehmen kann. Dies erlaubt es dem Operateur, Einfluss auf den Bewegungsumfang des Sägeblattes 28 im jeweiligen Führungsschlitz 32, 46 und damit relativ zum Grundkörper 18 zu nehmen.

Die Eintrittsöffnungen 64 sind beispielsweise quer zur Eingriffsrichtung 40 und längs der Eingriffsrichtung 40 angeordnet. Entsprechendes gilt für die Aufnahmen für die Knochenschrauben 58. Dementsprechend sind die Eintrittsöffnungen 64 am Grundkörper 18 beispielsweise "schachbrettförmig" angeordnet.

Durch Auswahl einer von der Aufnahme 60 unterschiedlichen Aufnahme für die Knochenschraube 58 zum Einnehmen der Blockierstellung kann insbesondere auch Einfluss auf den Winkel genommen werden, mit dem das Sägeblatt 28 in die Führungsschlitze 32, 46 eingreifen kann. Dies gibt die Möglichkeit, über die Knochenschraube 58 zu beeinflussen, in welchem Umfang der Femur 14 präpariert werden kann.

Die Eintrittsöffnungen 64 sind in Figur 1 schematisch nur nahe der Seitenfläche 24 dargestellt. Es ist selbstverständlich denkbar, dass vergleichbare Eintrittsöffnungen sowie die entsprechenden Aufnahmen ebenfalls nahe der Seitenfläche 25 angeordnet sind, für das jeweils andere Blockierelement 56.

Figur 1 zeigt nahe der Seitenfläche 25 schematisch eine andersartige Variante, bei der die Knochenschraube 58 relativ zum Grundkörper 18 unterschiedliche Blockierstellungen einnehmen könnte. Es ist gestrichelt die Eintrittsöffnung 66 einer Aufnahme für die Knochenschraube 58 dargestellt, die sich längs der Eingriffsrichtung 40 und quer zu dieser erstreckt. Diese Aufnahme ist nicht gezeigt. Die Eintrittsöffnung 66 ist L-förmig. In der entsprechenden Aufnahme ist die Knochenschraube 58 beweglich festlegbar, so dass sie längs der Eingriffsrichtung und quer zu dieser bewegt und an unterschiedlichen Positionen relativ zum Grundkörper 18 fixiert werden kann. Dies gibt ebenfalls die Möglichkeit, über die Knochenschraube 58 in einer gewählten Blockierstellung Einfluss auf den Bewegungsumfang des Sägeblattes 28 zu nehmen.

Es könnte selbstverständlich vorgesehen sein, dass eine zur Eintrittsöffnung 66 korrespondierend ausgestaltete Eintrittsöffnung sowie die entsprechende Aufnahme nahe der Seitenfläche 24 angeordnet ist.

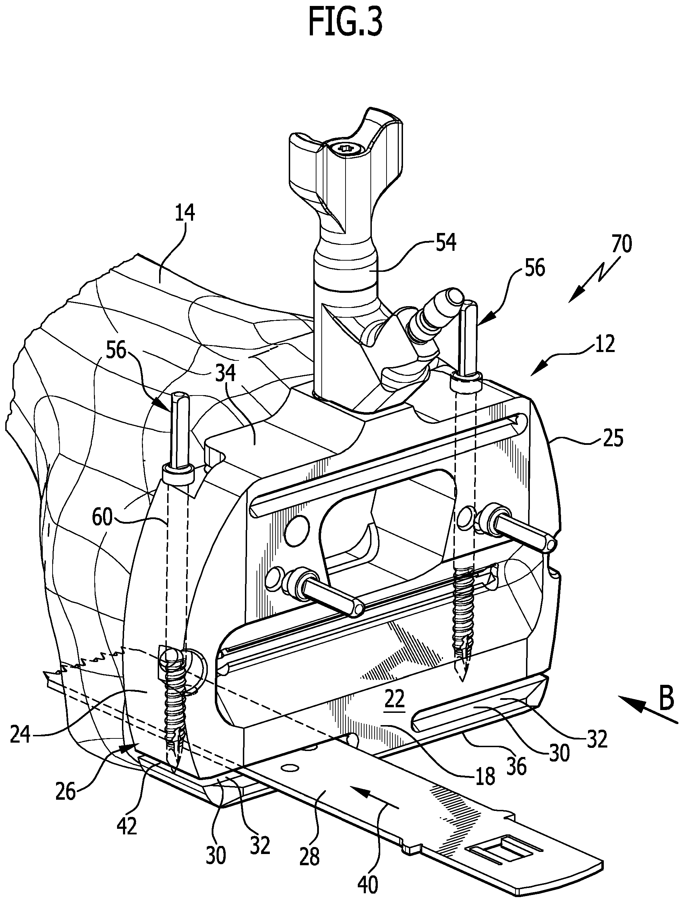

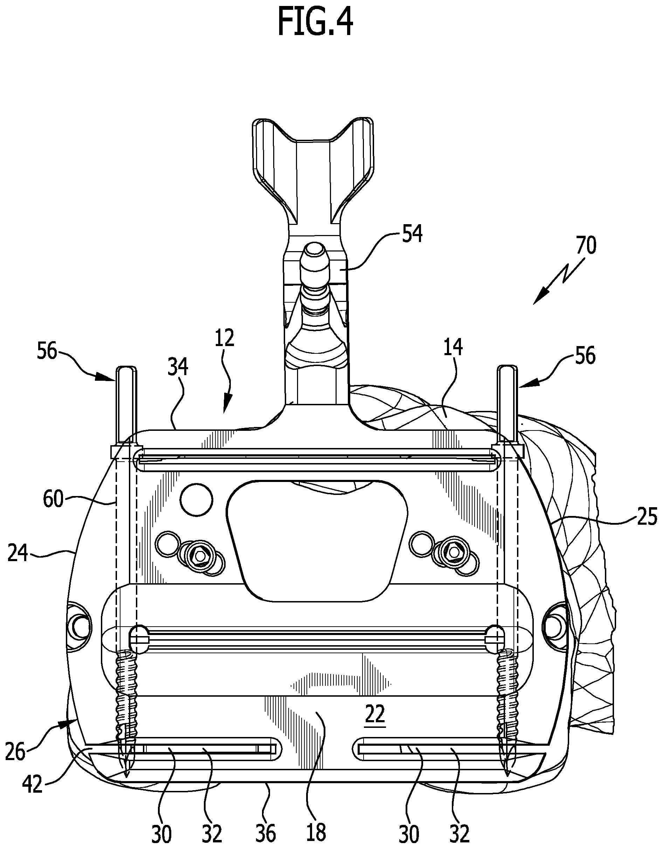

Die Figuren 3 und 4 zeigen eine weitere bevorzugte Ausführungsform eines erfindungsgemäßen Instrumentes, das mit dem Bezugszeichen 70 belegt ist. Das Instrument 70 umfasst einen Grundkörper 18, in dem nahe der unteren Seite 36 zwei Führungselemente 30 in Gestalt von Führungsschlitzen 32 mit seitlichen Öffnungen 42 an den Seitenflächen 24 bzw. 25 gebildet sind.

Die mit dem Instrument 10 erzielbaren Vorteile können mit dem Instrument 70 ebenfalls erzielt werden, so dass diesbezüglich auf die voranstehenden Erläuterungen verwiesen werden kann.

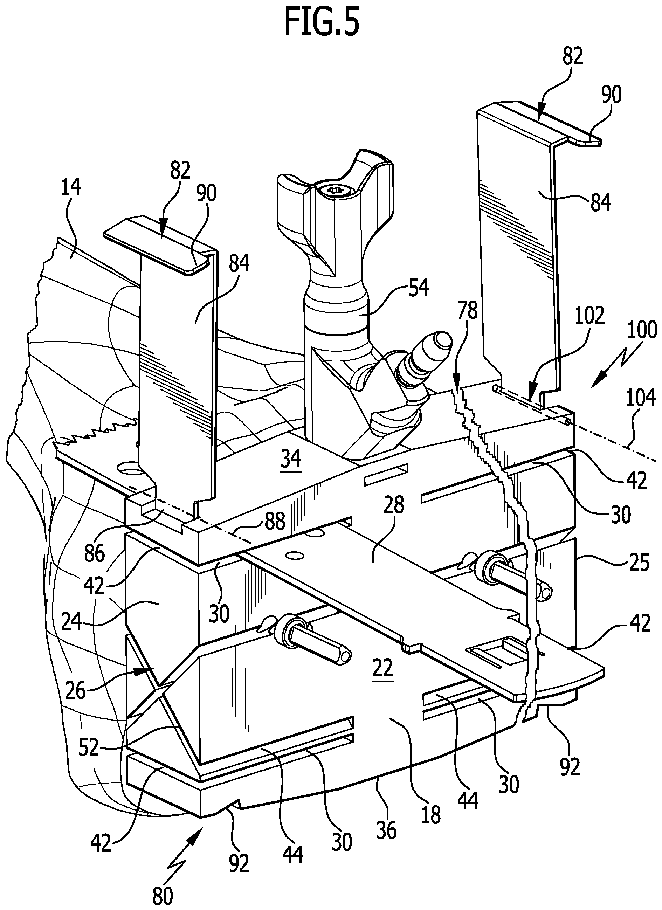

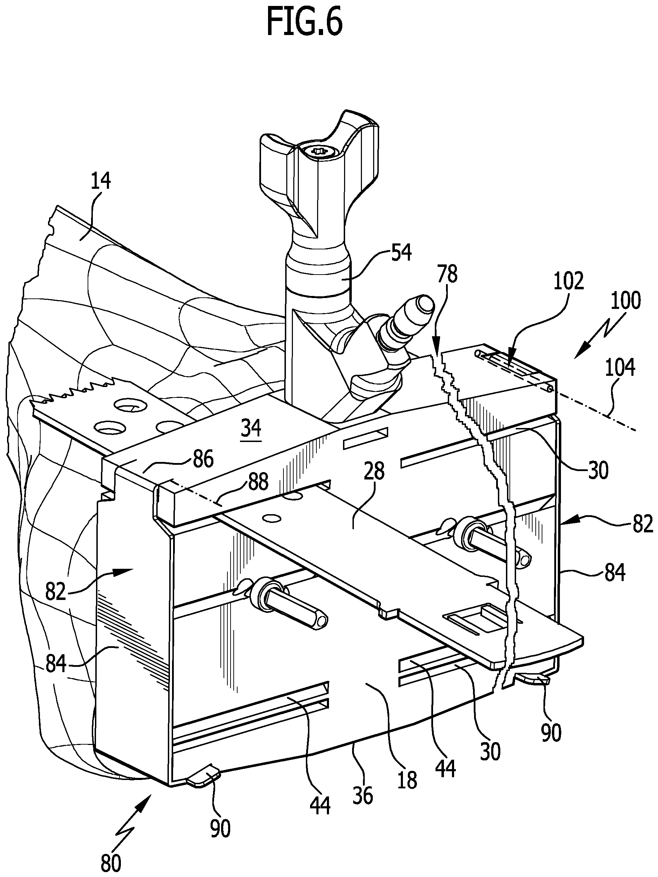

Nachfolgend wird auf die Figuren 5 und 6 eingegangen. In den Figuren 5 und 6 sind jeweils zwei vorteilhafte Ausführungsformen eines erfindungsgemäßen Instrumentes in einer gemeinsamen Figur dargestellt, eine jeweilige Unterbrechung 76 trennt die beiden mit den Bezugszeichen 80 bzw. 100 bezeichneten Ausführungsformen voneinander.

Beide Instrumente 80, 100 umfassen den Grundkörper 18, der weitgehend identisch ausgestaltet ist zum Grundkörper 18 des Instrumentes 10. Anders als beim Instrument 10 kommen jedoch nicht die Blockierelemente 56 in Gestalt der Knochenschrauben 58 zum Einsatz. Stattdessen sind nachfolgend erläuterte andersartige Blockierelemente vorgesehen.

Beim Instrument 80 kommt ein Blockierelement 82 zum Einsatz, das eine Abdeckung 84 umfasst. Die Abdeckung 84 ist am Grundkörper 18 festgelegt, vorzugsweise einstückig und insbesondere über ein Filmscharnier 86.

Das Filmscharnier 86 ist beispielsweise an der oberen Seite 34 angeordnet, nahe der Seitenfläche 24. Über das Filmscharnier 86 kann die Abdeckung 84 um eine parallel zur Eingriffsrichtung 40 ausgerichtete Schwenkachse 88 verschwenkt werden. Die Schwenkachse 88 ist parallel zur Eingriffsrichtung 40 ausgerichtet.

In einer Freigabestellung (Figur 5) sind die seitlichen Öffnungen 42, 52 der Führungsschlitze 32, 46 vom Blockierelement 82 freigegeben. Das Sägeblatt 28 kann am Rand 26 aus den Führungsschlitzen 32, 46 austreten.

Der Operateur kann das Blockierelement 82 durch Verschwenken um die Schwenkachse 88 in eine Blockierstellung überführen. In der Blockierstellung wirkt die Abdeckung 84 als Anschlag für das Sägeblatt 28 und verhindert dessen Austreten über die seitlichen Öffnungen 42, 52 aus den Führungsschlitzen 32 bzw. 46. Insbesondere überdeckt die Abdeckung 84 die seitlichen Öffnungen 42, 52 in der Blockierstellung des Blockierelementes 82 (Figur 6). Die Abdeckung 84 liegt vorzugsweise am Rand 26 an.

In der Blockierstellung kann das Blockierelement 82 am Grundkörper 18 fixiert werden. Beispielsweise ist eine Verrastung mit dem Grundkörper 18 über korrespondierende Rastelemente 90, 92 möglich. Bei dem Rastelement 90 handelt es sich zum Beispiel um einen Vorsprung, der an der Abdeckung 84 festgelegt ist. Das Rastelement 92 ist zum Beispiel eine Rastaufnahme, insbesondere an der unteren Seite 36.

Es ist für den Operateur auch möglich, das Blockierelement 82 ausgehend von der Blockierstellung in die Freigabestellung zu überführen. Hierzu kann die Verrastung der Rastelemente 90, 92 aufgehoben und das Blockierelement 82 um die Schwenkachse 88 verschwenkt werden.

Je nach Bedarf ist dadurch für den Operateur benutzerfreundlich die Möglichkeit gegeben zu entscheiden, ob er sich eines Blockierelementes 82 bedienen möchte oder nicht.

Obwohl das Blockierelement 82 vorstehend nur als an der Seitenfläche 24 angeordnet erläutert und gezeigt wurde, kann selbstverständlich vorgesehen sein, dass ein korrespondierend dazu ausgebildetes Blockierelement auch an der Seitenfläche 25 angeordnet ist, um die seitlichen Öffnungen 42, 52 an der Seitenfläche 25 des Randes 26 zu überdecken, wenn das Blockierelement die Blockierstellung einnimmt.

Das Instrument 100 ist weitestgehend identisch aufgebaut wie das Instrument 80. Der Unterschied zwischen den Instrumenten 80, 100 besteht darin, dass beim Instrument 100 Blockierelemente 82 vorgesehen sind, die mit dem Grundkörper 18 über ein Scharnier 102 schwenkbar verbunden sind. Das Scharnier 102 ist beim Instrument 100 kein Filmscharnier, sondern über ein Gelenk zwischen dem Blockierelement 82 und dem Grundkörper 18 gebildet. Das Scharnier 102 definiert eine Schwenkachse 104 zum Verschwenken des Blockierelementes 82, welche der Schwenkachse 88 des Instrumentes 80 entspricht. Die Schwenkachse 104 verläuft parallel zur Eingriffsrichtung 40.

Auch beim Instrument 100 kann das Blockierelement 82 von einer Freigabestellung in eine Blockierstellung und umgekehrt verschwenkt werden. In der Blockierstellung werden die seitlichen Öffnungen 42, 52 der Führungsschlitze 32 bzw. 46 überdeckt, in der Freigabestellung sind sie freigegeben.

Auch beim Instrument 100 kann das Blockierelement 82 in der Blockierstellung am Grundkörper 18 fixiert werden, zum Beispiel über die Rastelemente 90, 92.

Obwohl die Zeichnung die Anordnung des Blockierelementes 82 beim Instrument 100 nur an der Seitenfläche 25 zeigt, ist selbstverständlich denkbar, dass ein entsprechendes Blockierelement 82 auch an der Seitenfläche 24 angeordnet sein kann und in der Blockierstellung die an dieser Seite angeordneten Öffnungen 42, 52 überdecken kann.

### Bezugszeichenliste:

- 10: Instrument
- 12: Sägeblock
- 14: Femur
- 16: Fixierelement
- 18: Grundkörper
- 20: erste Seite
- 22: zweite Seite
- 24: Seitenfläche
- 25: Seitenfläche
- 26: Rand
- 28: Sägeblatt
- 30: Führungselement
- 32: Führungsschlitz
- 34: obere Seite
- 36: untere Seite
- 38: mittlerer Bereich
- 40: Eingriffsrichtung
- 42: seitliche Öffnung
- 44: Führungselement
- 46: Führungsschlitz
- 48: Eingriffsrichtung
- 52: seitliche Öffnung
- 54: Halteelement
- 56: Blockierelement
- 58: Knochenschraube
- 60: Aufnahme
- 62: Kanal/Sackloch
- 64: Eintrittsöffnung
- 66: Eintrittsöffnung
- 70: Instrument
- 78: Unterbrechung
- 80: Instrument
- 82: Blockierelement
- 84: Abdeckung
- 86: Filmscharmier
- 88: Schwenkachse
- 90: Rastelement
- 92: Rastelement
- 100: Instrument
- 102: Scharnier
- 104: Schwenkachse

## Patentansprüche

1. Chirurgisches Instrument zur Werkzeugführung, umfassend einen an einem zu bearbeitenden Körperteil (14) positionierbaren Grundkörper (18), der einstückig gebildet ist und einen Rand (26) mit einander gegenüberliegenden Seitenflächen (24, 25) aufweist, wobei am Grundkörper (18) Führungselemente (30, 44) zum Führen von einem in dieses in einer Eingriffsrichtung (40, 48) einführbaren chirurgischen Werkzeug (28) gebildet sind, wobei sich ein jeweiliges Führungselement (30, 44) im Winkel und insbesondere quer zur Eingriffsrichtung (40, 48) von einer Seitenfläche (24, 25) in Richtung der jeweils gegenüberliegenden Seitenfläche (24, 25) erstreckt und das jeweilige Führungselement (30, 44) am Rand (26) seitlich geöffnet ist, wobei die jeweils in einer gemeinsamen Ebene verlaufenden Führungselemente (30, 44) nicht miteinander verbunden sind oder nicht ineinander münden, wobei das Instrument (10; 70; 80; 100) mindestens ein am Grundkörper (18) in einer Blockierstellung festlegbares oder festgelegtes Blockierelement (56; 82) umfasst, das einen Anschlag für das Werkzeug (28) gegen Austreten aus einem der Führungselemente (30, 44) an der seitlichen Öffnung (45, 52) bildet.

2. Instrument nach Anspruch 1, **dadurch gekennzeichnet, dass** das Instrument (10; 70; 80; 100) ein chirurgischer Sägeblock (12) ist.

3. Instrument nach Anspruch 1 oder 2, **dadurch gekennzeichnet, dass** mindestens ein Führungselement (30, 44) schlitzförmig ist.

4. Instrument nach einem der voranstehenden Ansprüche, **dadurch gekennzeichnet, dass** der Grundkörper (18) aus einem Kunststoffmaterial gefertigt ist oder als Kunststoffformteil gebildet ist.

5. Instrument nach einem der voranstehenden Ansprüche, **dadurch gekennzeichnet, dass** das mindestens eine Blockierelement (56; 82) einen jeweiligen Anschlag an zwei oder mehr Führungselementen (30, 44) bildet.

6. Instrument nach einem der voranstehenden Ansprüche, **dadurch gekennzeichnet, dass** das mindestens eine Blockierelement (56; 82) mindestens ein Führungselement (30, 44) in der Blockierstellung durchsetzt.

7. Instrument nach einem der voranstehenden Ansprüche, **dadurch gekennzeichnet, dass** das mindestens eine Blockierelement (56, 82) stiftförmig, plattenförmig oder streifenförmig ausgestaltet ist.

8. Instrument nach einem der voranstehenden Ansprüche, **dadurch gekennzeichnet, dass** das mindestens eine Blockierelement (56) als Schraube ausgestaltet ist, insbesondere als Knochenschraube (58).

9. Instrument nach einem der voranstehenden Ansprüche, **dadurch gekennzeichnet, dass** das mindestens eine Blockierelement (56) in der Blockierstellung im Abstand zum Rand (26) angeordnet ist.

10. Instrument nach einem der Ansprüche 1 bis 8, **dadurch gekennzeichnet, dass** das mindestens eine Blockierelement (82) die seitliche Öffnung (45, 52) mindestens eines Führungselementes (30, 44) in der Blockierstellung zumindest teilweise überdeckt.

11. Instrument nach einem der voranstehenden Ansprüche, **dadurch gekennzeichnet, dass** das mindestens eine Blockierelement (56) getrennt vom Grundkörper (18) gebildet ist und an diesem zur Einnahme der Blockierstellung festlegbar ist oder dass das mindestens eine Blockierelement (82) einstückig mit dem Grundkörper (18) verbunden ist, beispielsweise mittels eines Filmscharniers (86).

12. Instrument nach einem der voranstehenden Ansprüche, **dadurch gekennzeichnet, dass** das mindestens eine Blockierelement (56; 82) am Grundkörper (18) gehalten ist, insbesondere dass das mindestens eine Blockierelement (56; 82) in der Blockierstellung vormontiert ist.

13. Instrument nach einem der voranstehenden Ansprüche, **dadurch gekennzeichnet, dass** das mindestens eine Blockierelement (82) ausgehend von der Blockierstellung in eine Freigabestellung überführbar ist, in der die seitliche Öffnung (45, 52) für das Werkzeug (28) freigegeben ist.

14. Instrument nach einem der voranstehenden Ansprüche, **dadurch gekennzeichnet, dass** das mindestens eine Blockierelement (82) am Grundkörper (18) schwenkbar und/oder verschieblich gelagert ist und von einer Freigabestellung, in der die seitliche Öffnung (45, 52) für das Werkzeug (28) freigegeben ist, in die Blockierstellung überführbar ist.

15. Instrument nach einem der voranstehenden Ansprüche, **dadurch gekennzeichnet, dass** das mindestens eine Blockierelement (56) am Grundkörper (18) wahlweise in einer einer Mehrzahl von Blockierstellungen festlegbar ist.

## Claims

1. Surgical instrument for tool guidance, comprising a base body (18) which is positionable on a body part (14) to be worked on and which is formed as one piece and has a rim (26) with opposing side faces (24, 25), wherein formed on the base body (18) are guidance elements (30, 44) for guiding a surgical tool (28) which is insertible into said element in an engagement direction (40, 48), wherein a respective guidance element (30, 44) extends at an angle and in particular transversely to the engagement direction (40, 48) from a side face (24, 25) in the direction of the respective opposite side face (24, 25) and the respective guidance element (30, 44) is laterally open at the rim (26), wherein the respective guidance elements (30, 44) running in a common plane are not connected to each other or do not open into each other, wherein the instrument (10; 70; 80; 100) comprises at least one blocking element (56; 82) which is securable or secured on the base body (18) in a blocking position and which forms a stop for the tool (28) from escaping out of one of the guidance elements (30, 44) at the lateral opening (45, 52).

2. Instrument in accordance with Claim 1, **characterized in that** the instrument (10; 70; 80; 100) is a surgical cutting block (12).

3. Instrument in accordance with Claim 1 or 2, **characterized in that** at least one guidance element (30, 44) is slot-shaped.

4. Instrument in accordance with any one of the preceding Claims, **characterized in that** the base body (18) is made of a plastics material or is formed as a plastic molded part.

5. Instrument in accordance with any one of the preceding Claims, **characterized in that** the at least one blocking element (56; 82) forms a respective stop at two or more guidance elements (30, 44).

6. Instrument in accordance with any one of the preceding Claims, **characterized in that** the at least one blocking element (56; 82) passes through at least one guidance element (30, 44) in the blocking position.

7. Instrument in accordance with any one of the preceding Claims, **characterized in that** the at least one blocking element (56, 82) has a pin-shaped, plate-shaped, or strip-shaped configuration.

8. Instrument in accordance with any one of the preceding Claims, **characterized in that** the at least one blocking element (56) is configured as a screw, in particular as a bone screw (58).

9. Instrument in accordance with any one of the preceding Claims, **characterized in that** the at least one blocking element (56) in the blocking position is arranged at a distance from the rim (26).

10. Instrument in accordance with any one Claims 1 to 8, **characterized in that** the at least one blocking element (82) at least partially covers the lateral opening (45, 52) of at least one guidance element (30, 44) in the blocking position.

11. Instrument in accordance with any one of the preceding Claims, **characterized in that** the at least one blocking element (56) is formed separately from the base body (18) and is securable thereon for adopting the blocking position, or **in that** the at least one blocking element (82) is connected as one piece to the base body (18), for example by means of a film hinge (86).

12. Instrument in accordance with any one of the preceding Claims, **characterized in that** the at least one blocking element (56; 82) is held on the base body (18), in particular **in that** the at least one blocking element (56; 82) is preassembled in the blocking position.

13. Instrument in accordance with any one of the preceding Claims, **characterized in that** the at least one blocking element (82) is transferable starting from the blocking position into a release position in which the lateral opening (45, 52) is unblocked for the tool (28).

14. Instrument in accordance with any one of the preceding Claims, **characterized in that** the at least one blocking element (82) is pivotably and/or displaceably mounted on the base body (18) and is transferable from a release position, in which the lateral opening (45, 52) is unblocked for the tool (28), into the blocking position.

15. Instrument in accordance with any one of the preceding Claims, **characterized in that** the at least one blocking element (56) is selectively securable on the base body (18) in a multitude of blocking positions.

## Revendications

1. Instrument chirurgical pour le guidage d'outil, comprenant un corps de base (18), qui peut être positionné sur une partie corporelle (14) à traiter, et qui est réalisé d'un seul tenant et présente une bordure (26) avec des surfaces latérales (24, 25) mutuellement opposées, instrument
dans lequel sur le corps de base (18) sont formés des éléments de guidage (30, 44) pour guider un outil chirurgical (28) pouvant y être introduit dans une direction d'intervention (40, 48),
dans lequel un élément de guidage (30, 44) respectivement considéré s'étend angulairement et notamment transversalement à la direction d'intervention (40, 48), d'une surface latérale (24, 25) en direction de la surface latérale (24, 25) respectivement opposée, et l'élément de guidage (30, 44) respectivement considéré est ouvert latéralement sur la bordure (26), et dans lequel les éléments de guidage (30, 44) s'étendant respectivement dans un plan commun, ne sont pas reliés mutuellement ou bien ne débouchent pas les uns dans les autres,
l'instrument (10; 70; 80; 100) comportant au moins un élément de blocage (56; 82), qui est fixé ou peut être fixé sur le corps de base (18) dans une position de blocage, et qui forme une butée pour l'outil (28) à l'encontre de sa sortie hors de l'un des éléments de guidage (30, 44) au niveau de l'ouverture latérale (45, 52) .

2. Instrument selon la revendication 1, **caractérisé en ce que** l'instrument (10; 70; 80; 100) est un bloc de sciage chirurgical (12).

3. Instrument selon la revendication 1 ou 2, **caractérisé en ce qu'**au moins un élément de guidage (30, 44) est en forme de fente.

4. Instrument selon l'une des revendications précédentes, **caractérisé en ce que** le corps de base (18) est fabriqué en une matière plastique, ou bien est réalisé sous forme de pièce de matière plastique moulée.

5. Instrument selon l'une des revendications précédentes, **caractérisé en ce que** ledit au moins un élément de blocage (56; 82) forme une butée respective au niveau de deux éléments de guidage (30, 44) ou davantage.

6. Instrument selon l'une des revendications précédentes, **caractérisé en ce que** ledit au moins un élément de blocage (56; 82) traverse au moins un élément de guidage (30, 44) dans la position de blocage.

7. Instrument selon l'une des revendications précédentes, **caractérisé en ce que** ledit au moins un élément de blocage (56, 82) est d'une configuration en forme de broche, en forme de plaque ou en forme de bande.

8. Instrument selon l'une des revendications précédentes, **caractérisé en ce que** ledit au moins un élément de blocage (56) est réalisé sous forme de vis, notamment sous forme de vis à os (58).

9. Instrument selon l'une des revendications précédentes, **caractérisé en ce que** ledit au moins un élément de blocage (56) est agencé, dans la position de blocage, à distance de la bordure (26).

10. Instrument selon l'une des revendications 1 à 8, **caractérisé en ce que** ledit au moins un élément de blocage (82) recouvre au moins partiellement l'ouverture latérale (45, 52) d'au moins un élément de guidage (30, 44), dans la position de blocage.

11. Instrument selon l'une des revendications précédentes, **caractérisé en ce que** ledit au moins un élément de blocage (56) est réalisé séparément du corps de base (18) et peut être fixé à celui-ci pour prendre la position de blocage, ou bien ledit au moins un élément de blocage (82) est relié d'un seul tenant au corps de base (18), par exemple au moyen d'une charnière à film (86).

12. Instrument selon l'une des revendications précédentes, **caractérisé en ce que** ledit au moins un élément de blocage (56; 82) est maintenu sur le corps de base (18), notamment **en ce que** ledit au moins un élément de blocage (56; 82) est prémonté dans la position de blocage.

13. Instrument selon l'une des revendications précédentes, **caractérisé en ce que** ledit au moins un élément de blocage (82) peut être transféré, à partir de la position de blocage, à une position de dégagement dans laquelle l'ouverture latérale (45, 52) est dégagée pour l'outil (28).

14. Instrument selon l'une des revendications précédentes, **caractérisé en ce que** ledit au moins un élément de blocage (82) est monté pivotant et/ou coulissant sur le corps de base (18), et peut être transféré, à partir d'une position de dégagement dans laquelle l'ouverture latérale (45, 52) pour l'outil (28) est dégagée, à la position de blocage.

15. Instrument selon l'une des revendications précédentes, **caractérisé en ce que** ledit au moins un élément de blocage (56) peut être fixé sélectivement au corps de base (18) dans l'une d'une pluralité de positions de blocage.
